# EUROPEAN PATENT APPLICATION

(11) **EP 3 300 832 A1**
(43) Date of publication of application: **04.04.2018**
(21) Application number: 17808742.5
(22) Date of filing: 20.06.2017
(51) Int. Cl.: B23K 26/382, A61M 37/00, B23K 26/073, H01S 3/00, H01S 3/10

(54) **METRHOD FOR MANUFACTURING MICRONEEDLE AND DEVICE FOR MANUFACTURING MICRONEEDLE**

(30) Priority: 25.07.2016 JP 2016145586
(71) Applicant: Think-Lands Co., Ltd., Yokohama-city, Kanagawa 230-0046 (JP)
(72) Inventor: SHIGA Noriyasu, Yokohama-city Kanagawa 230-0046 (JP); OIKAWA Yoichi, Yokohama-city Kanagawa 230-0046 (JP); MIYAJI Kunio, Yokohama-city Kanagawa 230-0046 (JP); TOYODA Kohei, Yokohama-city Kanagawa 230-0046 (JP)
(74) Representative: Plougmann Vingtoft a/s
(86) International application number: PCT/JP2017/022689
(87) International publication number: WO 2018/020911

(57) **Abstract**

Provided is a microneedle manufacturing method and a microneedle manufacturing device which are capable of forming a hollow needle. A microneedle manufacturing method of the present invention includes: an optical vortex conversion step of converting a laser beam into optical vortex pulsed light having a helical polarization; a pointed structure forming step of forming a pointed structure on a workpiece to be processed by irradiation of the optical vortex pulsed light; and a pore forming step of forming a hole or pore in the pointed structure by irradiating the pointed structure with laser pulsed light. A spot size of the laser pulsed light is smaller than a spot size of the optical vortex pulsed light.

## Description

### Technical Field

The present invention is suitable for application in, for example, manufacturing a microneedle made of a bioabsorbable polymer using pulsed light of a circularly polarized optical vortex beam in which the rotation direction of circular polarization matches the rotation direction of an optical vortex laser beam.

### Background Art

In recent years, an optical vortex technique using a circularly polarized optical vortex beam in which the rotation direction of circular polarization matches the rotation direction of an optical vortex laser beam has been attracting attention in various fields. The circularly polarized optical vortex beam is a laser beam having a helical phase obtained by causing a circularly polarized laser beam to pass through a helical phase plate, and has a characteristic different from that of a typical circularly polarized laser beam.

A technique is proposed in which a projection (needle-like body) is generated on the surface of a workpiece by utilizing an ablation phenomenon in which the surface of the workpiece is evaporated by irradiation of the circularly polarized optical vortex beam onto the workpiece (e.g., refer to Patent Literature 1).

### Citation List

### Patent Literature

Patent Literature 1: Japanese Patent No. 5531261

### Summary of Invention

### Technical Problem

However, the optical vortex technique described above is merely used for formation of a pointed structure, and there is a demand for manufacturing a so-called hollow needle having a tubular shape for injecting a drug or the like, such as an injection needle, as a microneedle.

The present invention has been made to solve the above-mentioned problem, and an object of the present invention is to provide a microneedle manufacturing method and a microneedle manufacturing device which are capable of forming a hollow needle.

### Solution to Problem

To solve the above-mentioned problem, a microneedle manufacturing method according to the present invention incudes: an optical vortex conversion step of converting a laser beam into optical vortex pulsed light having a helical polarization; a pointed structure forming step of forming a pointed structure on a workpiece to be processed by irradiation of the optical vortex pulsed light; and a pore forming step of forming a hole or pore in the pointed structure by irradiating the pointed structure with laser pulsed light. A spot size of the laser pulsed light is smaller than a spot size of the optical vortex pulsed light.

A microneedle manufacturing method according to the present invention includes: laser output means for outputting a laser beam of pulsed light; pointed structure forming means for converting the laser beam into optical vortex pulsed light having a helical polarization, and irradiating a workpiece to be processed with the optical vortex pulsed light to form a pointed structure on the workpiece; and pore forming means for forming a hole or pore in the pointed structure by irradiating laser pulsed light onto the pointed structure. A spot size of the laser pulsed light is smaller than a spot size of the optical vortex pulsed light.

### Advantageous Effects of Invention

According to the present invention, it is possible to implement a microneedle manufacturing method and a microneedle manufacturing device which are capable of forming a hollow needle.

### Brief Description of Drawings

[Figure 1] Figure 1 is a block diagram illustrating a configuration of the present invention.
[Figure 2] Figure 2 is a block diagram illustrating a configuration of a microneedle manufacturing device according to a first embodiment.
[Figure 3] Figure 3 is a schematic diagram illustrating a configuration of a common light irradiation section according to the first embodiment.
[Figure 4] Figure 4 is a schematic diagram illustrating a specific configuration of the microneedle manufacturing device according to the first embodiment.
[Figure 5] Figure 5 is a schematic diagram illustrating an example (1) of formation of a pore.
[Figure 6] Figure 6 is a schematic diagram illustrating an example (2) of formation of a pore.
[Figure 7] Figure 7 is a schematic diagram illustrating a configuration of a common light irradiation section according to a second embodiment.
[Figure 8] Figure 8 is a block diagram illustrating a configuration of a light irradiation section according to a third embodiment.
[Figure 9] Figure 9 is a block diagram illustrating a configuration of a light irradiation section according to a fourth embodiment.
[Figure 10] Figure 10 is a flowchart illustrating a hollow needle formation processing procedure.
[Figure 11] Figure 11 is a 3D photograph of a pointed structure in an example.
[Figure 12] Figure 12 is a photograph of a hollow needle taken by a stereoscopic microscope in the example.

### Description of Embodiments

Modes for carrying out the present invention will be described below with reference to the drawing.

As described above, Patent Literature 1 describes a method for forming a pointed structure using an optical vortex technique. The present inventor has invented a technique for manufacturing a so-called hollow needle having a tubular shape by forming a pore for injecting a drug or the like, such as an injection needle, on a pointed structure after the pointed structure is formed using the optical vortex technique.

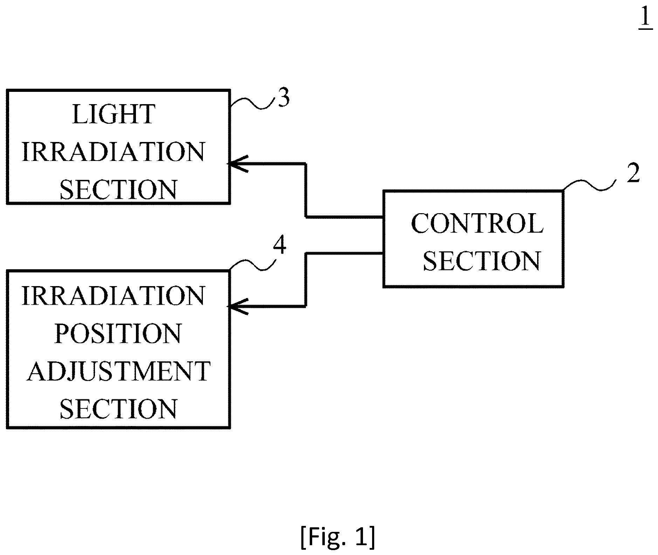

As illustrated in Figure 1, a microneedle manufacturing device 1 invented by the present inventor includes a control section 2 that controls the overall device, a light irradiation section 3 that irradiates light, and an irradiation position adjustment section 4 that adjusts an irradiation position. In the microneedle manufacturing device 1, after a pointed structure is formed by optical vortex pulsed light, a pore is formed in the pointed structure by circularly polarized laser pulsed light.

In a microneedle manufacturing device 1A according to first and second embodiments, the pointed structure and the pore are formed by a common light irradiation section 13 that irradiates both the optical vortex pulsed light and the laser pulsed light as the light irradiation section 3.

In a microneedle manufacturing device 1B according to the third embodiment, an irradiation position adjustment section 14 is commonly used, but the pointed structure and the pore are formed by separate light irradiation sections (a first light irradiation section 23A and a second light irradiation section 23B), respectively.

A microneedle manufacturing device 1C according to a fourth embodiment has a pointed structure forming device and a pore forming device (irradiation devices 1Ca and 1Cb), which are completely separate devices. The pointed structure is formed by the pointed structure forming device 1Ca, and the pore is formed by the pore forming device 1Cb.

A workpiece 9 having the pointed structure and the pore formed thereon is not particularly limited, but can be suitably used using, as major components, for example, metal such as aluminum and a stainless alloy, various inorganic materials such as mineral materials including silicon, carbon, ceramics, and calcium minerals, and organic high-molecular compounds. In particular, when the workpiece is used for medical and cosmetic uses, it is preferable to use a bioabsorbable material, which is harmless to a human body and absorbed in a living body, as a material for the workpiece 9.

A high-molecular compound described herein refers to an organic compound having a weight average molecular weight Mw of 5000 or more. A major component indicates that 50 weight% or more of high-molecular compound is contained in the entire workpiece. The high-molecular compounds preferably have a Tg (glass transition point) of 50°C or higher, and more preferably, 70°C or higher. This is because the high-molecular compound cannot be easily treated at room temperature when Tg is low.

As the high-molecular compound, only one type of high-molecular compound may be contained, or two or more types of high-molecular compound may be contained. Note that the ratio of the major component corresponds to the weight of the workpiece obtained after all processing steps for the workpiece are finished, and does not include a so-called solvent component to be intentionally evaporated in a drying process after formation of a fine projection. That is, the ratio of the major component is the ratio of the workpiece obtained after the processing for the workpiece is finished.

As the high-molecular compound, known compounds (synthetic polymer and naturally-occurring polymer) can be used. For example, various plastic materials, such as PET (polyethylene terephthalate), polyethylene, polypropylene, acrylic resin, epoxy resin, and polystyrene, as well as a bioabsorbable polymer, can be used.

As the bioabsorbable polymer, known compounds (synthetic polymer and naturally-occurring polymer) can be used. Examples of the bioabsorbable polymer include ester compounds such as polylactic acid, polyglycolic acid, poly-ε-caprolactone, poly-p-dioxane, and poly(malic acid), acid anhydride such as polyacid anhydride, orthoester compounds such as polyorthoester, carbonate compounds such as polycarbonate, phosphazene compounds such as poly(diaminophosphazene), peptide compounds such as synthetic polypeptide, phosphate ester compounds such as polyphosphoester urethane, carbon-carbon compounds such as polycyanoacrylate, poly-β-hydroxybutyric acid, ester compounds such as poly(malic acid), polyamino acids, chitin, chitosan, hyaluronic acid, sodium hyaluronate, pectic acid, galactan, starch, dextran, dextrin, alginic acid, sodium alginate, cellulose compounds (ethyl cellulose, carboxymethyl cellulose, hydroxy ethyl cellulose, hydroxypropyl cellulose, hydroxypropyl methyl cellulose, methyl cellulose), glycoside compounds (polysaccharides) such as gelatin, agar, Keltrol, Rheozan, xanthan gum, pullulan, and gum arabic, peptide compounds (peptide, protein) such as collagen, gelatin, fabrin, gluten, and serum albumin, phosphate ester compounds (nucleic acids) such as deoxyribonucleic acid and ribonucleic acid, and vinyl compounds such as polyvinyl alcohol.

The circularly polarized optical vortex beam used in the present invention is a circularly polarized laser beam having a helicity, and is pulsed light in which the rotation direction of circular polarization matches the rotation direction of an optical vortex laser beam. The pulse width of the pulsed light is appropriately selected depending on the material of the workpiece 9 and the size or the like of a small projection to be formed. The pulse width of the pulsed light is preferably equal to or more than 10 picoseconds and equal to or less than 100 nanoseconds.

As the circularly polarized optical vortex beam, a Laguerre-Gaussian beam, a Bessel-Gauss beam, and a multiplex optical vortex having a plurality of phase singularities at a wave front are illustrated. The Laguerre-Gaussian beam and the Bessel-Gauss beam have eigenmodes in cylindrical coordinates. The optical vortex laser beam becomes the Laguerre-Gaussian beam in a radius having a reflective index profile or a gain distribution proportional to the square of a radius vector, and becomes the Bessel-Gauss beam in a radius having no such a reflective index profile or gain distribution.

The Laguerre-Gaussian beam is a typical optical vortex laser beam, and an intensity distribution with respect to an optical axis cross-section at an intensity of zero (phase singularity) on the optical axis has a ring shape. In the Laguerre-Gaussian beam, the phase thereof changes by an integral multiple of 2π when the beam is rotated about the optical axis, like a spiral stairway, and an equiphase wave surface thereof has a helicity. This integer corresponds to the vortex order of the Laguerre-Gaussian beam. When the vortex order is a negative integer, the rotation direction is reversed.

Similarly to the Laguerre-Gaussian beam, in the Bessel-Gauss beam, the phase thereof changes by an integral multiple of 2π when the beam is rotated about the optical axis, like a spiral stairway, and an equiphase wave surface thereof has a helicity. This integer corresponds to the vortex order of the Bessel-Gauss beam. Examples of the multiplex optical vortex having a plurality of phase singularities at a wave front include a double optical vortex and a triple optical vortex. The double optical vortex has two phase singularities and two vortices, and the vortices have vortex orders of +1 order and -1 order, respectively. The triple optical vortex has three phase singularities and three vortices, and the vortices have vortex orders of +1 order, +1 order, and -1 order, respectively.

Specifically, the circularly polarized optical vortex beam is an optical vortex laser beam in which the spin angular momentum corresponding to the circular polarization is added to the orbital angular momentum corresponding to the vortex order of the optical vortex laser beam. In the circularly polarized optical vortex beam according to the present invention, the sign of the angular momentum of the orbital angular momentum corresponding to the vortex order of the optical vortex laser beam matches the sign of the angular momentum of the spin momentum corresponding to the circular polarization. In other words, the rotation direction of the optical vortex matches the rotation direction of the circular polarization. This is because, when the signs are opposite, i.e., when their rotation directions are opposed to each other, the orbital angular momentum of the optical vortex and the spin angular momentum of the circular polarization are cancelled out.

In a laser processing method and the microneedle manufacturing method according to the present invention, the method of generating the optical vortex laser beam is not particularly limited, examples of the method of generating the optical vortex laser beam include a method of generating the optical vortex laser beam by a fork-shaped hologram displayed on a liquid crystal spatial modulator, a method of generating the optical vortex laser beam by a helical phase plate, a method of generating the optical vortex laser beam by conversion from a Hermite-Gaussian mode, and a method of directly outputting the optical vortex laser beam from a laser resonator.

### <First Embodiment>

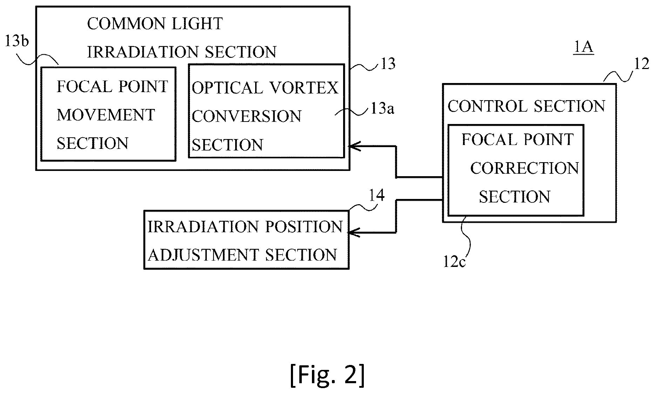

As illustrated in Figure 2, in the microneedle manufacturing device 1A, a control section 12 that is composed of an MPU (Micro Processing Unit), a ROM (Read Only Memory), and a RAM (Random Access Memory), which are not illustrated, controls the overall microneedle manufacturing device 1A in an integrated manner.

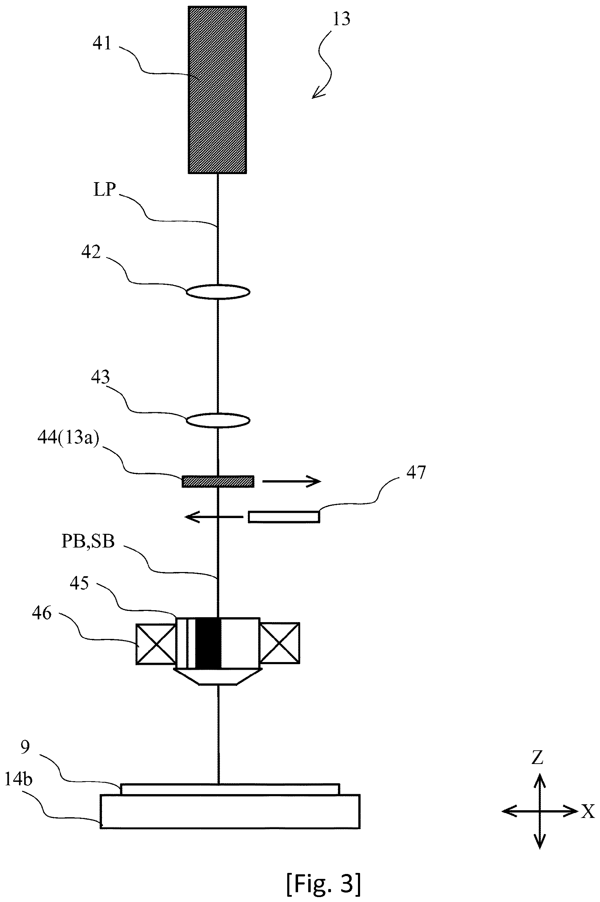

Figure 3 illustrates an optical system in the common light irradiation section 13. First, the formation of the pointed structure will be described.

A laser oscillator 41 is not particularly limited. In this example, the laser oscillator 41 is an Nd: YAG laser. The laser oscillator 41 oscillates pulsed light LP having a linear polarization using a Q switch. The pulse width of the pulsed light LP having a linear polarization is equal to or more than 10 picoseconds and equal to or less than 100 nanoseconds. When the pulse width is less than 10 picoseconds, plasma is less likely to be generated, and when the pulse width exceeds 100 nanoseconds, the problem of HAZ occurs. When the pulse width is 10 picoseconds or more, the light and the workpiece sufficiently interact with each other.

The wavelength of the pulsed light LP which has a linear polarization and is oscillated from the laser oscillator 41 is not particularly limited. However, when an inorganic material is used as the workpiece 9, a wavelength in a range from 2.0 µm to 0.2 µm at which the energy is large is suitably used. On the other hand, when a high-molecular compound is used as the workpiece 9, a wavelength of 1.0 µm or more or 10.0 µm or more is used. Unlike in the inorganic material, the high-molecular compound is decomposed due to overheat, and a carbonization phenomenon in which the high-molecular compound is carbonized occurs. Accordingly, when a laser light having a large energy and a short wavelength is used, the carbonization and ablation (sublimation) occur at the same time, which makes it extremely difficult to control the laser light. On the other hand, at a wavelength of 10.0 µm or more, the energy is extremely low and the efficiency deteriorates, and a minimum diameter at the time of light convergence is large, which makes it difficult to control the spot size. For reasons similar to those described above, when a high-molecular compound is used as the workpiece 9, the wavelength of the pulsed light LP that is equal to or more than 1.2 µm and less than 7.0 µm is suitably used.

The wavelength of the pulsed light LP is particularly preferably equal to or more than 1.5 µm and less than 4.0 µm. A so-called near-infrared region of 1.5 µm to 2.0 µm is preferable because a combination tone absorption of a hydrogen bond such as C-H, O-H, or N-H is observed, the absorbance is relatively small, and the equilibrium between the energy of the wavelength and the amount of absorption is preferably taken. In a so-called infrared region of 2.0 µm to 4.0 µm, an absorption specific to a polymeric organic compound is observed in many cases, the absorbance is extremely high, and ablation can be generated even at a low wavelength energy. In particular, a wavelength from 2.0 µm to 3.7 µm is preferable because an absorption band such as a broad hydroxyl group or amide group is present, and a slight blurring of the wavelength can be absorbed. Note that the wavelength of the pulsed light LP can be converted by, for example, a configuration of an optical parametric oscillation (OPO) using KTP crystal (KTiOPO₄), or by upconversion from a CO₂ laser.

In the laser processing method of the present invention, the output of the laser oscillator may be set so as to have a peak power density to be set, and is appropriately set depending on factors such as the spot diameter on the workpiece 9 of optical vortex pulsed light SB which is pulsed light of an optical vortex laser beam, the material of the workpiece 9 and the wavelength of the pulsed light LP. Preferably, the output of the laser oscillator is not particularly limited, but is preferably 0.01 mJ to 10 mJ. If the output is extremely small, the ablation does not occur or is insufficient, and if the output is extremely large, the carbonization of the workpiece 9 occurs. The spot diameter on the workpiece 9 of the optical vortex pulsed light SB is appropriately selected depending on the size of a small projection to be formed and not particularly limited, but is preferably 10 µm to 300 µm.

The pulsed light LP oscillated from the laser oscillator 41 and the size of a beam passing through a lens 42 having a focal length of 50 mm and a lens 43 having a focal length of 300 mm is increased to a six-fold, and the beam is converted into the optical vortex pulsed light SB by an optical vortex conversion section 13a which is a 12-divided helical phase plate 44. Note that the distance between the lens 42 having a focal length of 50 mm and the lens 43 having a focal length 300 mm is 350 mm. This is because the quality of the beam is improved due to the effective use of the area of the helical phase plate 44, and damage to the helical phase plate 44 is eliminated. The focal length is not particularly limited. After that, the beam is focused by an objective lens 45 (focal length 50 mm) and is irradiated onto the workpiece 9. The scale factor of the objective lens 45 is determined according to a desired spot diameter and is not particularly limited. In this example, the scale factor of the objective lens 45 is 5 to 50. The focal length of the objective lens 45 is not particularly limited.

The helical phase plate 44 is a phase plate having a thickness distribution controlled so as to provide a predetermined phase distribution with respect to a laser beam to be transmitted. The thickness distribution of the phase plate is approximated by a stepped discontinuous distribution, and the number of steps corresponds to the number of division. The number of division of the helical phase plate 44 is not particularly limited. For example, the helical phase plate 44 that is divided into 12 parts or 16 parts is used. Note that the optical vortex laser beam can be generated by the fork-shaped hologram displayed on the liquid crystal spatial modulator, instead of using the helical phase plate 44. The optical system is described in Patent Literature 1. Further, optical vortex oscillation devices described in Patent Literatures 3 to 5 may be appropriately applied.

Patent Literature 3: Japanese Patent No. 5831896
Patent Literature 4: Japanese Patent Application No. 2013-519522
Patent Literature 5: Japanese Patent No. 5035803

In the laser processing method of the present invention, the optical vortex laser beam is a Laguerre-Gaussian beam or a Bessel-Gauss beam. The vortex order is preferably an integer equal to or greater than 1, or an integer equal to or less than -1, and more preferably, the vortex order is an integer equal to or greater than 2, or an integer equal to or less than -2. As the vortex order of the Laguerre-Gaussian beam increases, the processed surface is more smoothed. A method for generating a Laguerre-Gaussian beam having a higher-order vortex order can be implemented by using helical phase plates in an overlapping manner. For example, the vortex order can be set to 2 by overlapping two helical phase plates used for generating a primary vortex. In a method for generating the optical vortex laser beam by the fork-shaped hologram displayed on the liquid crystal spatial modulator, the vortex order can be set to 2 by using a three fork-shaped hologram displayed on a phase plate liquid crystal spatial modulator. Further, in the laser processing method of the present invention, the optical vortex laser beam is preferably a multiplex optical vortex having a plurality of phase singularities at a wave front.

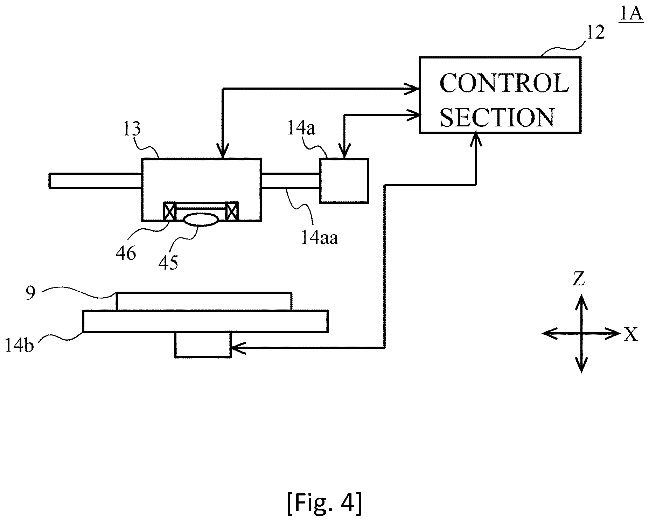

As illustrated in Figure 4, the optical path direction at the time of irradiation is referred to as a Z-direction, the direction of a slide rail 14aa of the thread motor 14a is referred to as an X-direction, and the direction perpendicular to each of the X-direction and the Z-direction is referred to as a Y-direction. The microneedle manufacturing device 1A includes: an actuator 46 that performs a fine adjustment in the X-, Y-, and Z-directions of the objective lens 45 as a focal point movement section 13b; the thread motor 14a that moves the objective lens 45 in the X-direction along the slide rail 14aa; and a stage 14b that adjusts the positions in the X-, Y-, and Z-directions of the workpiece 9 as the irradiation position adjustment section 14.

The control section 12 controls each of the focal point movement section 13b and the irradiation position adjustment section 14 to irradiate the workpiece 9 with the optical vortex pulsed light SB at a preliminarily set pointed structure forming interval, thereby forming a pointed structure. Although not illustrated, a light-receiving system in which return light from the surface of the workpiece 9 is split by a beam splitter or the like and the light is received by a photodetector or the like can be provided. The focal point position can be sequentially adjusted by the light-receiving system. Thus, the pointed structure can be reliably formed even when the surface of the workpiece 9 has irregularities.

In addition to this configuration, in the common light irradiation section 13, the pore is formed in the pointed structure by irradiating the pointed structure with the pulsed light LP as the laser pulsed light PB with usual linear polarization or circular polarization.

As illustrated in Figure 3, the common light irradiation section 13 is provided with the movable helical phase plate 44 as the optical vortex conversion section 13a. In the case of forming the pore, the helical phase plate 44 is disposed outside of the optical path.

In this case, for example, when a laser beam having a wavelength of 1.0 µm is used, a focus limit width is substantially the same as the wavelength of 1.0 µm. The spot size of the optical vortex pulsed light SB is set to a large value in a range from 10 to 300 µm, because the principle that the workpiece 9 that is sublimated to a central portion of the optical vortex is stacked is used.

If the laser pulsed light PB is irradiated with the same spot size, the laser pulsed light PB is irradiated so as to cover the formed pointed structure, so that the pointed structure is broken.

Accordingly, in the common light irradiation section 13, in the case of irradiating the laser pulsed light PB, the focused state of the laser pulsed light PB is changed when the laser pulsed light PB is incident on the objective lens 45 by the focal point movement section 13b, and the spot size (the diameter of the spot in the XY-direction) is reduced. Specifically, the common light irradiation section 13 is provided with a movable focused state change section 47. The focused state change section 47 is composed of a lens or diffraction grating for changing the focused state with respect to the objective lens 45. The focused state change section 47 is disposed outside of the optical path when the pointed structure is formed, while the focused state change section 47 is disposed on the optical path when the pore is formed.

The spot size at the time of irradiating the laser pulsed light PB is preferably about 1/3 to 1/20 of the optical vortex pulsed light SB, and more preferably, about 1/4 to 1/10 of the optical vortex pulsed light SB. An extremely large spot size is not preferable because the size of the pore becomes extremely large and the strength of the hollow needle becomes insufficient. An extremely small spot size is not preferable because the pore is extremely small and a space sufficiently large to inject a drug or the like cannot be ensured.

When the focused state of the laser pulsed light PB is changed when the laser pulsed light PB is incident on the objective lens 45, the focal point position in the X-direction is changed. Accordingly, the control section 12 drives the stage 14b in the Z-direction so that the focal point FP of the laser pulsed light PB matches a position in the vicinity of the top TP of the pointed structure.

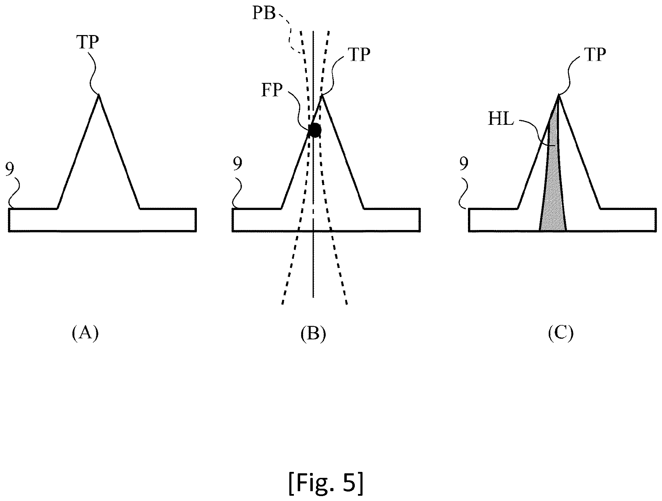

At this time, as illustrated in Figure 5, a pore HL can be formed at a position slightly (e.g., 5 to 20 µm) offset from the top TP of the pointed structure by shifting the position of the focal point FP in the XY-direction of the laser pulsed light PB from the top TP of the pointed structure. Thus, the pore HL can be formed while the sharpness of the pointed structure is maintained. The size of the offset (hereinafter referred to as a pore-center offset amount) is preferably the same as the radius of the pore HL or slightly larger than the radius of the pore HL (e.g., shifted to a larger extent by 0.5 to 5 µm from the radius of the pore HL). Thus, the pore HL can be formed without deforming the top of the pointed structure.

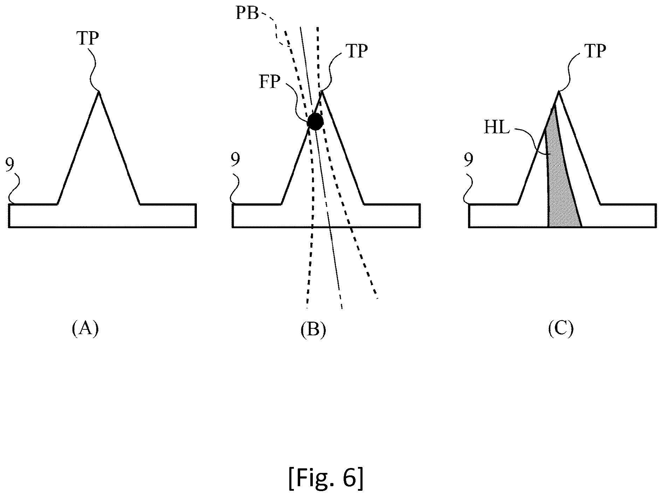

Further, as illustrated in Figure 6, the laser pulsed light PB is irradiated onto the workpiece 9 by slightly tilting the laser pulsed light PB from a perpendicular direction and the position of the focal point FP in the XY-direction of the laser pulsed light PB is shifted from the top TP of the pointed structure, thereby making it possible to form the pore HL that is substantially parallel to the tilt of the pointed structure. Thus, the area of the pore HL in the surface of the pointed structure can be minimized and the volume of the pore HL can be maximized while the strength of the pointed structure is maintained.

Specifically, in the case of forming the pointed structure, a focal point correction section 12a of the control section 12 controls the irradiation position adjustment section 14 to irradiate the workpiece 9 with the optical vortex pulsed light SB at a preliminarily set pointed structure forming position, thereby forming the pointed structure. Note that the workpiece 9 is placed in advance at a reference position on the stage 14b.

Next, the focal point correction section 12a irradiates the workpiece 9 with the laser pulsed light PB at the pore forming position, which is a position shifted from the pointed structure forming position by the pore-center offset amount, based on the pointed structure forming position, thereby forming the pore HL. At this time, in the control section 12, the focused state change section 47 is disposed on the optical path and the helical phase plate 44 is disposed outside of the optical path so that the focal point position in the Z-direction of the laser pulsed light PB is corrected. As a result, the pointed structure is appropriately irradiated with the laser pulsed light PB and thus the pore HL is formed. Note that the pore HL can be tilted as illustrated in Figure 6 by tilting the objective lens 45 by driving the actuator 46 (tilt is formed by greatly displacing only one side of the objective lens). Further, the focal point position in the Z-direction of the laser pulsed light PB is set in the vicinity of a slope of the pointed structure, so that a pore gradually spreading downward can be formed by using the characteristics of a Gaussian beam.

Note that the control section 12 controls the stage 14b, the thread motor 14a, and the actuator 46 to displace the focal point positions of the optical vortex pulsed light SB and the laser pulsed light PB to the pointed structure forming position and the pore forming position. However, any timing can be selected as a switch timing for forming the pointed structure and the pore. For example, the pore may be formed after one pointed structure is formed, or the pore may be formed in a row of pointed structures after the row of pointed structures is formed in the X-direction. Pores for a single sheet may also be formed after pointed structures for the single sheet is formed.

In this manner, in the microneedle manufacturing device 1A, the pointed structure is formed using the optical vortex pulsed light SB by the common light irradiation section 13, and then the pore is formed using the laser pulsed light PB by the common light irradiation section 13. Thus, the pore forming position may be determined based on the pointed structure forming position, which eliminates the need for detecting the position of the pointed structure. Further, since both the pointed structure and the pore can be formed by a single common light irradiation section 13, the configuration of the microneedle manufacturing device 1A can be simplified.

### <Examples>

Next, experimental results obtained when a hollow needle was actually formed will be described.

A hollow needle was created on the workpiece 9 by using the microneedle manufacturing device 1A. A sodium hyaluronate gel sheet containing purified water was used as the workpiece 9.

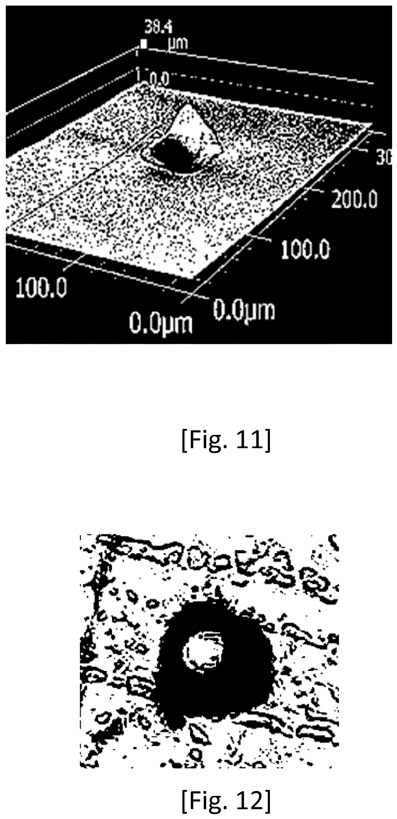

First, the pointed structure was formed by setting the focal point at the back of the surface of the workpiece 9 (inside of the workpiece). At this time, the power of the optical vortex pulsed light SB was 1.4 mj, the pulse width was 25 ps, the wavelength was 1.064 µm, and the spot size was about 20 µm at the focal point position. The pointed structure having a height of about 40 µm and a bottom surface diameter of about 80 µm was formed on the workpiece 9 as illustrated in Figure 11. Note that the spot size indicates the size (diameter) of a portion where the optical intensity decreases to an intensity of 13.5% (=1/e²) based on a maximum value.

Next, in an offset state where the center of the laser pulsed light PB is slightly shifted from the center of the pointed structure, the laser pulsed light PB of a normal Gaussian beam was irradiated by setting the initial focal point position in the vicinity of the surface of the pointed structure slightly offset from the top of the pointed structure, while the focal point position is slid to the inside of the pointed structure at a predetermined speed along with the formation of the pore. At this time, the power of the laser pulsed light PB was 0.1 mj, the pulse width was 25 ps, the wavelength was 1.064 µm, and the spot size was about 10 µm at the focal point position.

Figure 12 illustrates a microphotograph of the created hollow needle. A black portion indicates a pointed structure portion, and a white encircled portion indicates the formed pore. Thus, it was confirmed that a hollow needle can be manufactured using the same laser light source.

### <Second Embodiment>

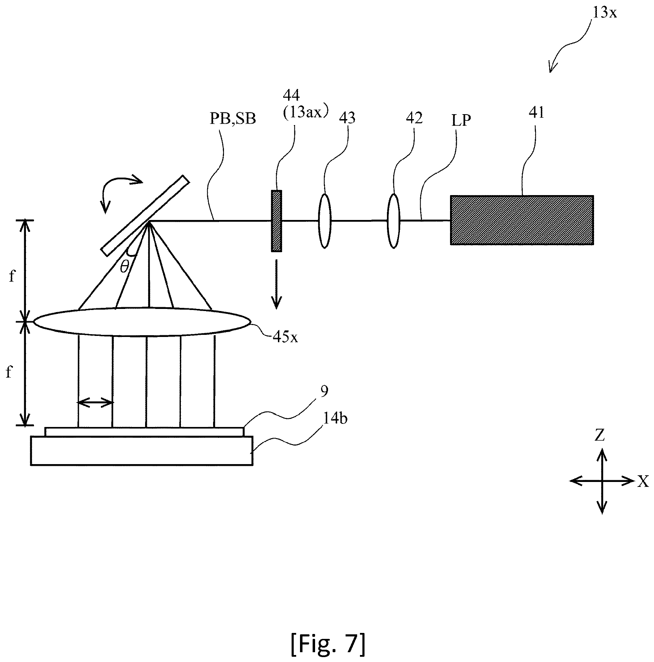

In a second embodiment illustrated in Figure 7, configurations of a common light irradiation section 13x and an irradiation position adjustment section 14x are partially different from those of the first embodiment. Note that parts of the second embodiment that correspond to those of the first embodiment are denoted by the same reference numerals, and descriptions thereof are omitted.

The common light irradiation section 13x in the microneedle manufacturing device 1Ax (not illustrated) includes, as an optical vortex conversion section 13ax, a liquid crystal type helical phase plate 44x that provides a phase difference by using light crystal. The liquid crystal type helical phase plate 44x is a fixed type that changes the display of the liquid crystal to thereby convert the pulsed light LP into the optical vortex pulsed light SB to be output, or output the pulsed light LP as the laser pulsed light PB with linear polarization or circular polarization. Therefore, the control section 12 changes the liquid crystal display of the liquid crystal type helical phase plate 44x, thereby making it possible to irradiate the workpiece 9 with the optical vortex pulsed light SB when the pointed structure is formed, and irradiate the workpiece 9 with the laser pulsed light PB when the pore is formed.

The common light irradiation section 13x also includes, as an irradiation position adjustment section 14ax, a rotatable galvanometer mirror 48, and uses a so-called Fθ lens that converts a spot moving in a focal point planar shape into a linear uniform motion by using a lens distortion effect as an objective lens 45x. Further, since the optical vortex pulsed light SB is irradiated in a direction perpendicular to a focal point plane (surface of the workpiece 9), a telecentric type is preferably used. Note that in the second embodiment, only the stage 14b is included as the irradiation position adjustment section 14ax, in addition to the galvanometer mirror 48.

Specifically, in the microneedle manufacturing device 1Ax, a laser scanning system including a combination of a fθ lens and a galvanometer mirror unit is adopted to two-dimensionally scan a laser beam and irradiate the workpiece 9 with the laser beam.

Further, in the microneedle manufacturing device 1Ax, when the pore is formed, the laser pulsed light PB is irradiated by decreasing the power to be lower than that when the pointed structure is formed, so that the size of the formed pore is set to be smaller than the diameter of the bottom surface of the pointed structure. Accordingly, the common light irradiation section 13x does not include the focused state change section 47 (Figure 3), unlike in the first embodiment.

Specifically, when the pointed structure is formed, the control section 12 controls the liquid crystal type helical phase plate 44x to convert the incident pulsed light LP into the optical vortex pulsed light SB. At this time, the focal point correction section 12a of the control section 12 controls the irradiation position adjustment section 14, and irradiates the workpiece 9 with the optical vortex pulsed light SB at a preliminarily set pointed structure forming position, thereby forming the pointed structure. Note that the workpiece 9 is placed in advance at the reference position on the stage 14b.

Next, the control section 12 controls the liquid crystal type helical phase plate 44x to form the pore, and outputs the incident pulsed light LP as the laser pulsed light PB. At this time, the focal point correction section 12a irradiates the workpiece 9 with the laser pulsed light PB at the pore forming position, which is a position shifted from the pointed structure forming position by the pore-center offset amount, based on the pointed structure forming position, thereby forming the pore HL.

The microneedle manufacturing device 1Ax forms a plurality of pointed structures and pores within an irradiation range of the galvanometer mirror unit (the galvanometer mirror 48 and the objective lens 45x), displaces the stage 14b in the XY-direction, and further forms a plurality of pointed structures and pores.

Thus, in the microneedle manufacturing device 1Ax, the use of the two-dimensional scanning system using the galvanometer mirror unit makes it possible to form a large number of pointed structures and pores rapidly.

Although not illustrated, instead of the galvanometer mirror 48, a diffraction grating may be used to divide one light beam into a plurality of light beams and irradiate the workpiece 9 with the light beams, thereby making it possible to form a plurality of pointed structures and a plurality of pores at the same time.

### <Third Embodiment>

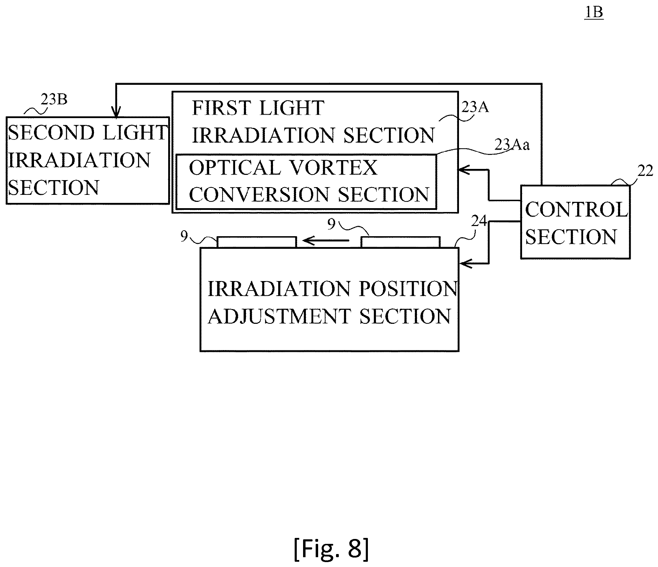

A third embodiment illustrated in Figure 8 differs from the first embodiment described above in that the formation of a pointed structure and the formation of a pore are separately executed by two irradiation sections having different configurations, though the irradiation position adjustment section 14 is commonly used. Note that parts of the third embodiment that correspond to those of the first embodiment are denoted by like reference numerals to which 10 is added, and descriptions of the same parts are omitted.

In the microneedle manufacturing device 1B according to the third embodiment, the irradiation position adjustment section 24 has a configuration in which the stage on which the workpiece 9 is placed is movable in the Y-direction. On the workpiece placed on the stage, the pointed structure is formed by the optical vortex pulsed light SB irradiated from the first light irradiation section 23A, and then the workpiece advances in the Y-direction (left direction in the figure) to form the pore by the laser pulsed light PB irradiated from the second light irradiation section 23B.

In the microneedle manufacturing device 1B, the two light irradiation sections 23A and 23B having different configurations irradiate the optical vortex pulsed light SB and the laser pulsed light PB, respectively, and thus the microneedle manufacturing device 1B may be composed of an optical system corresponding to the respective irradiation of the light. Specifically, the first light irradiation section 23A having a structure in which the helical phase plate 44 in the common light irradiation section 13 illustrated in Figure 3 is fixed onto the optical path and which does not include the focused state change section 47 is used. The common light irradiation section 13x illustrated in Figure 7 may also be used.

As the second light irradiation section 23B, a light irradiation section having a configuration in which the helical phase plate 44 and the focused state change section 47 are removed from the common light irradiation section 13 illustrated in Figure 3 is used. A configuration in which the liquid crystal type helical phase plate 44x is removed from the common light irradiation section 13x illustrated in Figure 7 may also be used.

Specifically, in the case of forming the pointed structure, the control section 12 irradiates the workpiece 9 with the optical vortex pulsed light SB at a preliminarily set pointed structure forming position to form the pointed structure. Note that the workpiece 9 is placed in advance at the reference position on the stage. Next, the control section 12 irradiates the workpiece 9 with the laser pulsed light PB at the pore forming position to form the pore.

The microneedle manufacturing device 1B can simultaneously form the pointed structure and the pore, which enables a high manufacturing efficiency to be attained and is thus suitable for mass production. Further, since the workpiece 9 placed on the stage is automatically moved, there is no need to perform alignment.

Note that the amount of movement of the workpiece 9 generated along with the movement of the stage is corrected in advance to adjust the pore forming position of the workpiece 9 in the second light irradiation section 23B to match the position of the pointed structure, without performing alignment. This adjustment is performed using an adjustment sample having, for example, a mark or the like for adjustment.

### <Fourth Embodiment>

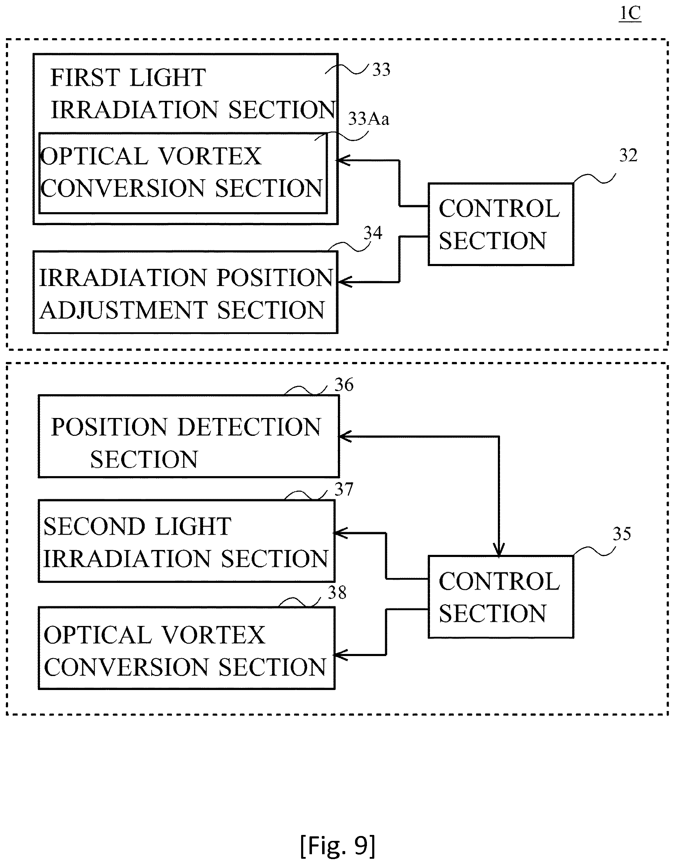

A fourth embodiment illustrated in Figure 9 differs from the third embodiment described above in that the formation of a pointed structure and the formation of a pore are executed by the two irradiation devices 1Ca and 1Cb having different configurations, respectively. Note that parts of the fourth embodiment that correspond to those of the third embodiment are denoted by like reference numerals to which 10 is added, and descriptions of the same parts are omitted.

As illustrated in Figure 9, in the microneedle manufacturing device 1C according to the fourth embodiment, the two irradiation devices 1Ca and 1Cb having different configurations form a pointed structure and a pore, respectively. The irradiation device 1Cb is provided with a position detection section 36, which detects the accurate position of the workpiece 9 based on the formed pointed structure, or the mark or the like for position adjustment that is formed in advance on the workpiece 9, and sets the pore forming position.

Thus, in the microneedle manufacturing device 1C, the two irradiation devices 1Ca and 1Cb having different configurations form the pointed structure and the pore, respectively, thereby making it possible to manufacture the microneedle.

### <Microneedle Preparation Process>

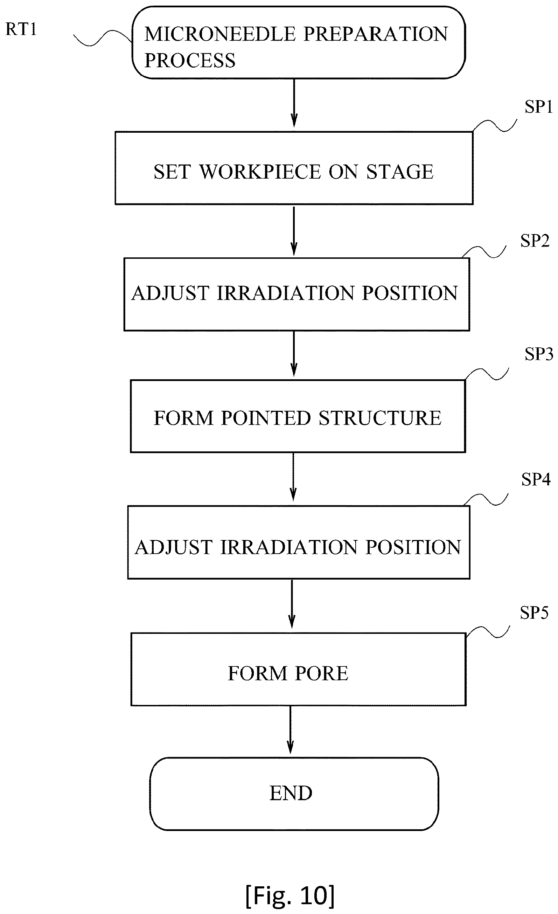

Next, a microneedle preparation process procedure RT1 will be described with reference to the flowchart of Figure 10.

In step SP1, the control section 12 recognizes that the workpiece 9 is set on the stage when, for example, a start button is operated by a person in charge of production, or an automatic detection function is activated, the process proceeds to the next step SP2.

In step SP2, the control section 12 sets the irradiation position to irradiate the optical vortex pulsed light SB at the pointed structure forming position, and then the process proceeds to the next step SP3. In step SP3, the control section 12 irradiates the workpiece 9 with the optical vortex pulsed light SB to form the pointed structure, and then the process proceeds to the next step SP4.

In step SP4, the control section 12 sets the irradiation position to irradiate the laser pulsed light PB at the pore forming position, and then the process proceeds to the next step SP5. In step SP5, the control section 12 irradiates the workpiece 9 with the laser pulsed light PB to form the pore, and then the process proceeds to an end step to terminate the process.

### <Inventions extracted from the embodiments described above>

Features of invention groups extracted from the embodiments described above will be described below while illustrating problems to be solved, advantageous effects, and the like, as needed. Note that in the following description, for ease of understanding, the components corresponding to those in the embodiments are illustrated, as needed, in parentheses or the like. However, the present invention is not limited to the specific components illustrated in parentheses or the like. The meaning, illustration, or the like of each term described in the features may be applied as the meaning, illustration, or the like of each term described in other described features with the same wording.

Heretofore, an optical vortex technique for irradiating a workpiece with the circularly polarized optical vortex beam to generate a projection (needle-like body) on the surface of the workpiece by using the ablation phenomenon in which the surface of the workpiece is evaporated has been proposed (e.g., refer to Patent Literature 1).

However, the optical vortex technique is used only to form a pointed structure, and it is necessary to form a pore in the pointed structure to manufacture a microneedle having a hollow needle shape.

As an invention for manufacturing a microneedle having a hollow needle shape, the following features can be extracted. An invention A group can be extracted in terms of being capable of manufacturing a microneedle having a hollow needle shape. Further, an invention B group can be extracted in terms of being capable of simplifying the configuration of the microneedle manufacturing device and easily performing alignment. Furthermore, an invention C group can be extracted in terms of being capable of easily performing alignment and efficiently forming a pointed structure and a pore.

### <Invention A Group>

Feature A1: A microneedle manufacturing method according to the present invention includes: an optical vortex conversion step of converting a laser beam (pulsed light LP) into optical vortex pulsed light (optical vortex pulsed light SB) having a helical polarization;
a pointed structure forming step of forming a pointed structure on a workpiece to be processed by irradiation of the optical vortex pulsed light; and
a pore forming step of forming a hole or pore (pore HL) in the pointed structure by irradiating the pointed structure with laser pulsed light (laser pulsed light PB). A spot size of the laser pulsed light is smaller than a spot size of the optical vortex pulsed light.

Note that the size of the spot (diameter) of the laser pulsed light is preferably about 1/2 to 1/10 of the spot size of the optical vortex pulsed light. If the spot size of the laser pulsed light is extremely large, the pointed structure may be broken, and thus an extremely large spot size is not preferable. On the other hand, if the spot size of the laser pulsed light is extremely small, the pore cannot be formed or is extremely small, and thus an extremely small spot size is not preferable.

According to the microneedle manufacturing method described in the feature A1, the pore can be formed on the pointed structure, which is formed using a laser beam, by using a laser beam, and thus the affinity between the step of forming the pointed structure and the step of forming the pore is high, and the hollow needle can be formed by accurately forming the pore in the pointed structure.

Feature A2: In the microneedle manufacturing method described in the feature A1, in the pore forming step,
an optical vortex focal point position, which is a focal point position of the optical vortex pulsed light on the workpiece, and a laser focal point position (focal point FP), which is a focal point position of the laser pulsed light on the workpiece, are displaced in an optical axis direction.

According to the microneedle manufacturing method described in the feature A2, in the case of forming the pointed structure in which the surface of the workpiece is flat, the optical vortex focal point position is set based on the plane, and the laser focal point position can be set based on the pointed structure at the time of forming the pore after the pointed structure is formed. Accordingly, an appropriate focal point position can be set in each process for forming the hollow needle.

Feature A3: In the microneedle manufacturing method described in the feature A1 or the feature A2,
in the pore forming step,
the pointed structure is irradiated with the laser pulsed light based on the laser beam.

According to the microneedle manufacturing method described in the feature A3, the optical vortex pulsed light and the laser pulsed light are generated based on the laser beam emitted from the same light source, and both the pointed structure and the pore can be formed by a single optical system. Therefore, the microneedle manufacturing device and the manufacturing process can be simplified.

Feature A4: In the microneedle manufacturing method described in any one of the features A1 to A3,
in the pore forming step,
an optical vortex focal point position, which is a focal point position of the optical vortex pulsed light on the workpiece, and a laser focal point position, which is a focal point position of the laser pulsed light on the workpiece, are displaced in a planar direction perpendicular to the optical axis direction.

According to the microneedle manufacturing method described in the feature A4, the top of the pointed structure and the center of the pore in the workpiece can be offset in the planar direction, and the hollow needle can be formed while the height of the top of the pointed structure is maintained. Further, changing the height of the circumferential portion of the pore makes it possible to reduce the area of the microneedle to be inserted into the flesh of a human when the microneedle is inserted into the skin of the human. Therefore, a needle which causes a little damage to a human body when the needle is used for injection and is human-body-friendly can be formed.

Feature A5: In the microneedle manufacturing method described in any one of the features A1 to A4,
in the pore forming step, an optical vortex focal point position, which is a focal point position of the optical vortex pulsed light on the workpiece, and a laser focal point position, which is a focal point position of the laser pulsed light on the workpiece, are displaced by tilting the optical vortex focal point position and the laser focal point position in the optical axis direction.

According to the microneedle manufacturing method described in the feature A5, the pore can be formed by tilting the pore with respect to the pointed structure, so that the pore can be formed along the conical pointed structure, the diameter of the pore can be increased with respect to the pointed structure, and the strength of a wall (a portion in which the pore is not formed) of the pointed structure can be increased.

Feature A6: In the microneedle manufacturing method described in any one of the features A1 to A5,
the laser focal point position is set in the vicinity of the tilted surface of the pointed structure. Note that the vicinity of the tilted surface refers to a position where the laser focal point position is located within a range of 1/5, and more preferably, 1/10, of the height of the pointed structure from the tilted surface. For example, when the pointed structure is 200 µm, the laser focal point position is located within a range of a distance of 40 µm, and more preferably, 20 µm, from the surface of the pointed structure. Note that if the pointed structures have various shapes, the laser focal point position is set based on the shape of an average pointed structure.

According to the microneedle manufacturing method described in the feature A6, a pore gradually spreading downward along the cone of the pointed structure can be formed using the shape of a Gaussian beam.

Feature A7: A microneedle manufacturing device (microneedle manufacturing device 1) according to the present invention includes:
a laser output means (laser oscillator 41) for outputting a laser beam of pulsed light;
a pointed structure forming means (common light irradiation section 13) for converting the laser beam into optical vortex pulsed light having a helical polarization, and irradiates a workpiece to be processed with the optical vortex pulsed light to form a pointed structure on the workpiece;
a pore forming means (common light irradiation section 13) for irradiating the pointed structure with laser pulsed light to form a hole or pore in the pointed structure. A spot size of the laser pulsed light is smaller than a spot size of the optical vortex pulsed light.

According to the microneedle manufacturing device described in the feature A7, since both the pointed structure and the pore can be formed using a laser, the manufacturing device can be designed using similar or identical optical systems, the configuration of the manufacturing device can be simplified, and the adjustment of the positions of the pointed structure and the pore can be simplified.

Feature A8: In the microneedle manufacturing device (microneedle manufacturing device 1) described in the feature A7,
the pointed structure forming means includes:
a displacement mirror section configured to displace an optical vortex focal point position of the optical vortex pulsed light in a planar direction by a movable mirror; and
an objective lens configured to focus the optical vortex pulsed light output from the displacement mirror section and irradiate the optical vortex pulsed light in a direction substantially perpendicular to the workpiece.

According to the microneedle manufacturing device described in the feature A8, pointed structures can be sequentially formed by a small amount of movement and a rapid movement of the displacement mirror section, which leads to a considerable improvement in the speed of manufacturing the microneedle.

Feature A9: In the microneedle manufacturing device described in the feature A7 or A8,
the pore forming means includes:
a displacement mirror section configured to displace the laser focal point position of the laser pulsed light in a planar direction by a movable mirror; and
an objective lens configured to focus the laser pulsed light output from the displacement mirror section, and irradiate the laser pulsed light in a direction substantially perpendicular to the workpiece.

According to the microneedle manufacturing device described in the feature A9, pores can be sequentially formed by a small amount of movement and a rapid movement of the displacement mirror section, which leads to a considerable improvement in the speed of manufacturing the microneedle.

Feature A10: In the microneedle manufacturing device described in the feature A7,
the pointed structure forming means includes:
a divider configured to divide the optical vortex pulsed light to be distributed to a plurality of optical vortex focal point positions in a planar direction; and
an objective lens configured to focus the optical vortex pulsed light output from the divider and irradiate the optical vortex pulsed light in a direction substantially perpendicular to the workpiece.

According to the microneedle manufacturing device described in the feature A10, the optical vortex pulsed light can be divided into a plurality of light beams by the divider, thereby making it possible to simultaneously form a plurality of pointed structures and considerably improve the speed of manufacturing the microneedle.

Feature A11: In the microneedle manufacturing device described in the feature A7 or A10,
the pointed structure forming means includes:
a divider configured to divide the optical vortex pulsed light to be distributed to a plurality of optical vortex focal point positions in a planar direction; and
an objective lens configured to focus the laser pulsed light output from the divider and irradiate the laser pulsed light in a direction substantially perpendicular to the workpiece.

In the microneedle manufacturing device described in the feature A11, the optical vortex pulsed light is divided into a plurality of light beams by the divider, thereby making it possible to simultaneously form a plurality of pores and considerably improve the speed of manufacturing the microneedle.

Feature A12: In the microneedle manufacturing device described in any one of the features A7 to A11,
the pointed structure forming means and the pore forming means each include:
laser output means for outputting pulsed light of a laser beam;
an objective lens configured to focus pulsed light based on the laser beam and irradiate a workpiece to be processed with the pulsed light;
selective optical vortex conversion means disposed between the laser output means and the objective lens, the selective optical vortex conversion means being capable of causing the laser beam to pass therethrough as laser pulsed light, while converting the laser beam into optical vortex pulsed light having a helical polarization; and
a control section configured to control the selective optical vortex conversion means.
The workpiece is irradiated with the optical vortex pulsed light and the laser pulsed light from the same optical path.

According to the microneedle manufacturing device described in the feature A12, the use of the same optical system makes it possible to reduce a time required for position adjustment and further improve the speed of manufacturing the microneedle.

### <Invention B group>

Feature B1: A microneedle manufacturing method according to the present invention includes: an optical vortex conversion step of converting a laser beam (pulsed light LP) into optical vortex pulsed light having a helical polarization;
a pointed structure forming step of forming a pointed structure on a workpiece (workpiece 9) to be processed by irradiation of the optical vortex pulsed light; and
a pore forming step of forming a hole or pore in the pointed structure by irradiating the pointed structure with laser pulsed light. In the pore forming step, the pointed structure is irradiated with the laser pulsed light based on the laser beam.

According to the invention described in the feature B1, the hole or pore can be formed in the pointed structure by irradiating the workpiece with the laser beam emitted from a single light source as the optical vortex pulsed light and the laser pulsed light. Consequently, a hollow needle having a homogeneous configuration can be formed by well using the same characteristics.

Feature B2: in the microneedle manufacturing method described in the feature B1,
in the pore forming step,
the workpiece is irradiated with laser pulsed light through the same objective lens as that for the optical vortex pulsed light, and the laser pulsed light is irradiated at a laser focal point position based on an optical vortex focal point position which is a focal point position of the optical vortex pulsed light on the workpiece.

According to the microneedle manufacturing method described in the feature B2, the laser pulsed light is irradiated at the laser focal point position based on the optical vortex focal point position through the same objective lens. Accordingly, the laser pulsed light can be accurately irradiated at an assumed position on the formed pointed structure, without the need for detecting the position of the pointed structure and performing a detailed position adjustment, and the hollow needle can be formed as is simulated.

Feature B3: In the microneedle manufacturing method described in any one of the feature B2,
in the pointed structure forming step,
the workpiece is placed on object mount means (irradiation position adjustment section 24) capable of displacing the workpiece at least in a planar direction perpendicular to a laser traveling direction, and
in the pore forming step,
a focal point of the optical vortex pulsed light and a focal point of the laser pulsed light are slightly displaced in the planar direction in such a manner that the laser pulsed light is irradiated at an offset position slightly offset from an optical vortex irradiation position at which the optical vortex pulsed light is irradiated in the pointed structure forming step, by moving the object mount means.

According to the microneedle manufacturing method described in the feature B3, the irradiation position of the laser pulsed light can be adjusted at the offset position slightly offset from the optical vortex irradiation position merely by moving the object mount means, which eliminates the need for fine adjustment of the irradiation position.

Feature B4: In the microneedle manufacturing method described in any one of the features B1 to B3,
in the pore forming step,
the laser pulsed light is slightly tilted with respect to the workpiece, and the laser focal point position in the optical axis direction is located in the vicinity of the surface of the pointed structure.

According to the microneedle manufacturing method described in the feature B4, a pore gradually spreading downward from the surface of the pointed structure can be formed by using the shape of a Gaussian beam, and a pore having a large diameter can be formed while the strength of the wall of the pointed structure is maintained.

Feature B5: Microneedle manufacturing devices (microneedle manufacturing devices 1A and 1B) according to the present invention include:
laser output means for outputting pulsed light of a laser beam;
an objective lens configured to focus pulsed light based on the laser beam and irradiate a workpiece to be processed with the pulsed light;
selective optical vortex conversion means disposed between the laser output means and the objective lens, the selective optical vortex conversion means being capable of causing the laser beam to pass therethrough as laser pulsed light, while converting the laser beam into optical vortex pulsed light having a helical polarization; and
a control section configured to control the selective optical vortex conversion means.

According to the microneedle manufacturing device described in the feature B5, both the optical vortex pulsed light and the laser pulsed light can be irradiated using a single optical system, so that the configuration of the microneedle manufacturing device can be simplified. The laser pulsed light can be irradiated at the same position merely by irradiating the laser pulsed light at the position where the pointed structure is formed, thereby substantially eliminating the need for fine position adjustment.

Feature B6: The microneedle manufacturing device described in the feature B5 includes object mount means on which the workpiece is placed, the object mount means being configured to be displaced at least in a planar direction perpendicular to a laser traveling direction. The control section controls the object mount means to irradiate the laser pulsed light at an offset position at which a focal point of the laser pulsed light is slightly offset in the planar direction from an optical vortex irradiation position at which the optical vortex pulsed light is irradiated.

According to the microneedle manufacturing device described in the feature B6, the irradiation position can be easily adjusted by moving the object mount means, which eliminates the need for a configuration for specifying the position of the pointed structure.

Feature B7: In the microneedle manufacturing device described in the feature B5, the selective optical vortex conversion means converts the pulsed light into the optical vortex pulsed light by a conversion element configured to convert the pulsed light into an optical vortex, and the selective optical vortex conversion means outputs the pulsed light as the laser pulsed light by preventing the pulsed light from passing through the conversion element.

According to the microneedle manufacturing device described in the feature B7, the optical vortex pulsed light and the laser pulsed light can be switched with a simple configuration of allowing the pulsed light to pass through the conversion element, or preventing the pulsed light from passing through the conversion element.

Feature B8: In the microneedle manufacturing device described in any one of the features B1 to B7, the selective optical vortex conversion means is a conversion element having a variable pattern capable of adjusting the polarization of the pulsed light by changing a polarization pattern,
the selective optical vortex conversion means converts the pulsed light into the optical vortex pulsed light and outputs the optical vortex pulsed light with a polarization pattern for converting the pulsed light into an optical vortex, and
the selective optical vortex conversion means outputs the pulsed light as the laser pulsed light with a polarization pattern for preventing a polarization direction of the pulsed light from being changed.

According to the microneedle manufacturing device described in the feature B8, the optical vortex pulsed light and the laser pulsed light can be switched with a simple configuration by switching the polarization patterns.

Feature B9: The microneedle manufacturing device described in any one of the features B5 to B7 includes lens drive means capable of displacing the objective lens at least in the optical axis direction. The control section displaces an optical vortex focal point position, which is a focal point position of the optical vortex pulsed light on the workpiece, and a laser focal point position, which is a focal point position of the laser pulsed light on the workpiece, in the optical axis direction by controlling the lens drive means.

According to the microneedle manufacturing device described in the feature B9, the optical vortex focal point position and the laser focal point position can be displaced to irradiate the optical vortex pulsed light and the laser pulsed light at an appropriate focal point position with a simple configuration of merely controlling the lens drive means.

Feature B10: in the microneedle manufacturing device described in the feature B9, the control section controls the lens drive means to irradiate the laser pulsed light at an offset position at which the focal point of the laser pulsed light is slightly offset in the planar direction from the optical vortex irradiation position at which the optical vortex pulsed light is irradiated.

According to the microneedle manufacturing device described in the feature B10, the optical vortex focal point position and the laser focal point position can be displaced to irradiate the optical vortex pulsed light and the laser pulsed light at an appropriate focal point position with a simple configuration of merely controlling the lens drive means.

Feature C1: The needle manufacturing device according to the present invention includes:
object mount means on which a workpiece to be processed is placed;
pointed structure forming means for converting a laser beam of pulsed light into optical vortex pulsed light having a helical polarization, and irradiating the optical vortex pulsed light at the set optical vortex focal point position, thereby forming the pointed structure on the workpiece;
pore forming means for forming a hole or pore in the pointed structure by irradiating the laser pulsed light at a set laser focal point position; and
an irradiation control section configured to set the laser focal point position based on the optical vortex focal point position.

According to the microneedle manufacturing device described in the feature C1, the laser focal point position can be set based on the optical vortex focal point position, and the adjustment of the laser focal point position on the pointed structure can be simplified.

Feature C2: In the microneedle manufacturing device described in the feature C1, the pointed structure forming means includes:
a laser output section configured to output the laser beam;
an optical vortex conversion section configured to convert the laser beam into optical vortex pulsed light; and
an objective lens configured to focus the optical vortex pulsed light and irradiate the workpiece with the optical vortex pulsed light, and
the pore forming means irradiates the workpiece with the laser pulsed light based on the laser beam through the objective lens.

According to the microneedle manufacturing device described in the feature C2, since the pointed structure forming means and the pore forming means use a common objective lens, the laser pulsed light is irradiated, for example, under the same conditions for the optical vortex pulsed light, and the optical vortex focal point position and the laser focal point position can be set at the same position, and thus the laser focal point position can be easily adjusted based on the optical vortex focal point position.

Feature C3: In the microneedle manufacturing device described in the feature C1 or the feature C2,
the optical vortex conversion section is disposed between the laser output means and the objective lens, and is a selective optical vortex conversion section capable of causing the laser beam to pass therethrough as laser pulsed light, while converting the laser beam into optical vortex pulsed light having a helical polarization.

According to the microneedle manufacturing device described in the feature C3, the pointed structure and the pore or hole can be formed by a single optical system, and the configuration of the microneedle manufacturing device can be simplified.

### (Common optical system + displacement in the optical axis direction according to the focused state)

Feature C4: In the microneedle manufacturing device described in any one of the features C1 to C3,
the pore forming means includes a focused state change section configured to change the focused state of the laser pulsed light to be incident on the objective lens, and
the object mount means is displaced in the optical axis direction according to the displacement of the laser focal point position in the optical axis direction due to a change in the focused state.

According to the microneedle manufacturing device described in the feature C4, the pointed structure and the pore or hole having a diameter smaller than that of the pointed structure can be formed for a short period of time by a single optical system, and the configuration of the microneedle manufacturing device can be simplified.

Feature C5: In the microneedle manufacturing device described in the feature C1,
the pointed structure forming means and the pore forming means have different configurations, and the pointed structure forming means and the pore forming means irradiate the workpiece, which is placed on the object mount means commonly used, with the optical vortex pulsed light and the laser pulsed light.

According to the microneedle manufacturing device described in the feature C5, since the object mount means is commonly used, the laser focal point position can be set based on the optical vortex focal point position.

Feature C6: In the microneedle manufacturing device described in the feature C3,
the pointed structure forming means and the pore forming means have different configurations, and
the microneedle manufacturing device further includes a switching section capable of switching a laser to be irradiated between the optical vortex pulsed light and the laser pulsed light.

According to the microneedle manufacturing device described in the feature C6, the optical vortex pulsed light and the laser pulsed light can be irradiated at substantially the same position by switching between the optical vortex pulsed light and the laser pulsed light.

Feature C7: In the microneedle manufacturing device described in any one of the features C1 to C6, the object mount means displaces the workpiece within an irradiation range of the optical vortex pulsed light and the laser pulsed light.

Note that the phrase "within an irradiation range" refers to a range in which the workpiece can be appropriately irradiated with the optical vortex pulsed light and the laser pulsed light by adjusting the positions of the objective lens and the optical system.

According to the microneedle manufacturing device described in the feature C7, the laser pulsed light can be irradiated at any position on the pointed structure merely by correcting the difference between the positional relationship between the pointed structure forming means and the optical vortex irradiation position and the positional relationship between the pore forming means and the laser irradiation position on the workpiece, and by arbitrarily setting an offset or the like.

Feature C8: In the microneedle manufacturing device described in the feature C7,
the irradiation control section simultaneously executes the formation of the pointed structure and the formation of the pore by the optical vortex pulsed light and the laser pulsed light.

According to the microneedle manufacturing device described in the feature C8, both the formation of the pointed structure and the formation of the pore can be simultaneously performed, which leads to a considerable improvement in production efficiency.

Feature C9: In the microneedle manufacturing device described in the feature C7 or C8,
the irradiation control section sets the laser focal point position based on the optical vortex focal point position by correcting the difference between an optical vortex relative positional relationship between the formed pointed structure and the objective lens of the pointed structure forming means when the object mount means is within the irradiation range of the optical vortex pulsed light, and a laser relative positional relationship between the pointed structure and the objective lens of the pore forming means when the workpiece is displaced into the irradiation range of the laser pulsed light.

According to the microneedle manufacturing device described in the feature C9, in the case of forming the pore, there is no need to detect the position of the workpiece, which facilitates the position adjustment.

### <Invention D Group>

Feature D1: A microneedle manufacturing method according to the present invention includes:
an optical vortex conversion step of converting a laser beam into optical vortex pulsed light having a helical polarization;
a pointed structure forming step of forming a pointed structure on a workpiece to be processed by irradiation of the optical vortex pulsed light; and
a pore forming step of forming a hole or pore in the pointed structure by irradiating the pointed structure with laser pulsed light.

In the pore forming step,
a top of the pointed structure is detected, and a laser focal point position, which is a focal point position of the laser pulsed light on the workpiece is determined based on the top of the pointed structure.

According to the microneedle manufacturing method described in the feature D1, the laser pulsed light can be irradiated at any position on the pointed structure, and the hole or pore can be formed in the pointed structure.

Feature D2: The microneedle manufacturing method described in the feature D1 includes:
laser output means for outputting pulsed light of a laser beam;
an objective lens configured to focus circularly-polarized laser pulsed light based on the laser beam and irradiate a workpiece to be processed with the laser pulsed light;
detection means for detecting a top of a pointed structure on the workpiece;
focal point position adjustment means for adjusting a focal point position of the laser pulsed light on the workpiece; and
a control section configured to control the focal point position adjustment means so as to irradiate the laser pulsed light at an irradiation position based on a position of the top detected by the detection means.

According to the microneedle manufacturing method described in the feature D2, the laser pulsed light can be irradiated at any position on the pointed structure, and thus the hole or pore can be formed in the pointed structure.

### Industrial Applicability

The present invention can be applied to, for example, a microneedle to be used as an injection needle for medical or cosmetic applications.

### Reference Signs List

1, 1A, 1Ax, 1B, 1C: Microneedle manufacturing device
1Ca: Pointed structure forming device
1Cb: Pore forming device
2: Control section
3: Light irradiation section
4: Irradiation position adjustment section
9: Workpiece
12: Control section
12a: Focal point correction section
13, 13x: Common light irradiation section
13a, 13ax: Optical vortex conversion section
13b: Focal point movement section
14, 24: Irradiation position adjustment section
14a: Thread motor
14aa: Slide rail
14ax: Irradiation position adjustment section
14b: Stage
14x: Irradiation position adjustment section
23A: First light irradiation section
23B: Second light irradiation section
41: Laser oscillator
42, 43: Lens
44: Helical phase plate
44x: Liquid crystal type helical phase plate
45, 45x: Objective lens
FP: Focal point
HL: Pore
LP: Pulsed light
PB: laser pulsed light
RT1: Preparation process procedure
SB: Optical vortex pulsed light
TP: Top

## Claims

1. A microneedle manufacturing method comprising:
an optical vortex conversion step of converting a laser beam into optical vortex pulsed light having a helical polarization;
a pointed structure forming step of forming a pointed structure on a workpiece to be processed by irradiation of the optical vortex pulsed light; and
a pore forming step of forming a hole or pore in the pointed structure by irradiating the pointed structure with laser pulsed light,
wherein a spot size of the laser pulsed light is smaller than a spot size of the optical vortex pulsed light.

2. The microneedle manufacturing method according to Claim 1, wherein in the pore forming step, an optical vortex focal point position and a laser focal point position are displaced in an optical axis direction, the optical vortex focal point position being a focal point position of the optical vortex pulsed light on the workpiece, the laser focal point position being a focal point position of the laser pulsed light on the workpiece.

3. The microneedle manufacturing method according to Claim 1 or 2, wherein in the pore forming step, the pointed structure is irradiated with the laser pulsed light based on the laser beam.

4. The microneedle manufacturing method according to any one of Claims 1 to 3, wherein an optical vortex focal point position and a laser focal point position are displaced in a planar direction perpendicular to an optical axis direction, the optical vortex focal point position being a focal point position of the optical vortex pulsed light on the workpiece, the laser focal point position being a focal point position of the laser pulsed light on the workpiece.

5. The microneedle manufacturing method according to any one of Claims 1 to 4, wherein in the pore forming step, an optical vortex focal point position and a laser focal point position are displaced by slightly tilting the optical vortex focal point position and the laser focal point position from the optical axis direction, the optical vortex focal point position being a focal point position of the optical vortex pulsed light on the workpiece, the laser focal point position being a focal point position of the laser pulsed light on the workpiece.

6. The microneedle manufacturing method according to any one of Claims 1 to 5, wherein the laser focal point position is set in a vicinity of a tilted surface of the pointed structure.

7. A microneedle manufacturing device comprising:
laser output means for outputting a laser beam of pulsed light;
pointed structure forming means for converting the laser beam into optical vortex pulsed light having a helical polarization, and irradiating a workpiece to be processed with the optical vortex pulsed light to form a pointed structure on the workpiece; and
pore forming means for forming a hole or pore in the pointed structure by irradiating laser pulsed light onto the pointed structure,
wherein a spot size of the laser pulsed light is smaller than a spot size of the optical vortex pulsed light.

8. The microneedle manufacturing device according to Claim 7, wherein the pointed structure forming means includes:
a displacement mirror section configured to displace an optical vortex focal point position of the optical vortex pulsed light in a planar direction using a movable mirror; and
an objective lens configured to focus the optical vortex pulsed light output from the displacement mirror section and irradiate the optical vortex pulsed light in a direction substantially perpendicular to the workpiece.

9. The microneedle manufacturing device according to Claim 7 or 8, wherein the pore forming means includes:
a displacement mirror section configured to displace a laser focal point position of the laser pulsed light in a planar direction using a movable mirror; and
an objective lens configured to focus the laser pulsed light output from the displacement mirror section and irradiate the laser pulsed light in a direction substantially perpendicular to a workpiece.

10. The microneedle manufacturing device according to Claim 7, wherein the pointed structure forming means includes:
a divider configured to divide the optical vortex pulsed light to be distributed to a plurality of optical vortex focal point positions in a planar direction; and
an objective lens configured to focus the optical vortex pulsed light output from the divider and irradiate the optical vortex pulsed light in a direction substantially perpendicular to the workpiece.

11. The microneedle manufacturing device according to Claim 7 or 10, wherein the pointed structure forming means includes:
a divider configured to divide the laser pulsed light to be distributed to a plurality of laser focal point positions in a planar direction; and
an objective lens configured to focus the laser pulsed light output from the divider and irradiate the laser pulsed light in a direction substantially perpendicular to a workpiece.

12. The microneedle manufacturing device according to any one of Claims 7 to 11, wherein the pointed structure forming means and the pore forming means each include:
laser output means for outputting pulsed light of a laser beam;
an objective lens configured to focus the pulsed light based on the laser beam and irradiate the workpiece to be processed with the pulsed light;
selective optical vortex conversion means disposed between the laser output means and the objective lens, the selective optical vortex conversion means being capable of causing the laser beam to pass therethrough as laser pulsed light, while converting the laser beam into optical vortex pulsed light having a helical polarization; and
a control section configured to control the selective optical vortex conversion means,
wherein the workpiece is irradiated with the optical vortex pulsed light and the laser pulsed light from the same optical path.

13. The microneedle manufacturing device according to any one of Claims 7 to 12, further comprising:
object mount means on which a workpiece to be processed is placed; and
an irradiation control section configured to set a laser focal point position being a focal point position of the laser pulsed light based on an optical vortex focal point position being a focal point position of the optical vortex pulsed light.

14. The microneedle manufacturing device according to Claim 13, wherein the pointed structure forming means includes:
a laser output section configured to output the laser beam;
an optical vortex conversion section configured to convert the laser beam into optical vortex pulsed light; and
an objective lens configured to focus the optical vortex pulsed light and irradiate the workpiece with the optical vortex pulsed light,
wherein the pore forming means irradiates the workpiece with the laser pulsed light based on the laser beam as the laser pulsed light through the objective lens.

15. The microneedle manufacturing device according to Claim 14, wherein the optical vortex conversion section is a selective optical vortex conversion section disposed between the laser output means and the objective lens, the selective optical vortex conversion section being capable of causing the laser beam to pass therethrough as laser pulsed light, while converting the laser beam into optical vortex pulsed light having a helical polarization.
